# EUROPEAN PATENT APPLICATION

(11) **EP 2 135 597 A1**
(43) Date of publication of application: **23.12.2009**
(21) Application number: 09161070.9
(22) Date of filing: 26.05.2009
(51) Int. Cl.: A61K 8/02, A61K 8/39, A61K 8/92, A61Q 5/06

(54) **A high water content lip stick and its preparation**

(30) Priority: 20.06.2008 CN 200810125700
(71) Applicant: Evonik Goldschmidt GmbH, 45127 Essen (DE)
(72) Inventor: Zhang, Haizhou, 201108, Shanghai (CN); Dai, Jingya, 201108, Shanghai (CN); Zou, Jiali, Minhang District, Shanghai (CN); Gao, Zhiheng, 201108, Shanghai (CN)

(57) **Abstract**

This invention describes a high water content lip stick and its preparation. This formula includes 1-10 wt% emulsifiers, 1-30 wt% water phase and 69-98 wt% oil components phase. Pigments, pearl agents, water soluble active ingredients can be introduced when required. Polyglyceryl fatty ester, fatty ester and silicone derivative are combined as the mixed emulsifier to form a water-in-oil pre emulsion and then add this pre emulsion into other oil components. This preparing method is easy to handle and can increase the water content effectively. The samples produced according to this invention are very stable in storage and usage: no coarse surface or air bubbles phenomena appear. As this kind of formula comprises water, the cost may be lowered comparing with normal anhydrous formulas, moreover, water soluble active ingredients are also possible to be added, which will give the consumer a totally new feeling. The invention can be widely applied in lip care products.

## Description

### Field of the Invention

The present invention relates to a high water content lipstick and its preparation, which is suitable to be applied in personal care.

### Background

Normally, lipstick or lip balm formulas are anhydrous, thus water soluble active ingredients which have skin caring effect are difficult to be added. Although some products in the market contain a certain content of water, the water content is always less than 5 wt% (% by weight).

CN1260166A relates to a W/O emulsion, which contains at least 25 wt% water phase, at least a silicone emulsifier and at least 5 wt% wax. Microcristalline wax is also included. The oil phase containing kinds of wax is 20 wt% - 75 wt%. And the silicone emulsifier could be ABIL^{®} WE 09. The composition also contains Propylene glycol as moisture agent. Chinese patent application CN1260166A describes high water content (higher than 25 wt%) cream formulas, but these formulas can not keep its shape stable in storage or application, therefore they are not suitable to be used as lipstick formulas.

A lot of references mention that ABIL^{®} EM 90 could be used as the emulsifier of W/O system, wherein the water could includes polyols, e.g. CN1260166A, CN1251773A, CN1199608A, CN1592603A, CN1373654A. None of these documents describe the use of ABIL® WE 09 to produce lipsticks.

CN1328817A refers to a stable W/O stick composition, which contains 0.5 wt% - 40 wt% hydrogened Jojoba oil, 0.10 wt% - 10.0 wt% surfactant and 5 wt% - 50 wt% water. The surfactant is a polyoxyalkylene-modified silicone.

### Detailed description of the invention

This invention provides a method to produce high water content in order to solve the problem of low water content and difficult to add water active ingredients in normal lipstick formulas nowadays. The high water content makes the formula cost lowered and gives the consumer a softer, comfortable and caring feeling when applied.

The lip stick described in this invention is a kind of product for decoration and/or caring in lip area, when demoded, they can be stable from -15 °C to 45 °C for a certain period of time: keep its stick shape and show no oil separation, crack, small holes or bubbles. Furthermore, it can also keep its shape when applied. The invention can be lip sticks of all kinds e. g. with pigments or decorative materials, with caring ingredients (without pigments or colorants) or with a transparent or translucent appearance.

This invention provides a high water content lip stick formula which comprises from 1 wt% to 10 wt% emulsifiers, from 1 wt% to 30 wt%, preferably 10 wt% to 30 wt% and more preferably from 17 wt% to 25 wt% of a water phase and from 69 wt% to 98 wt%, preferably 75 wt% to 95 wt% of an oil components phase, wherein the proportion of pure water of the whole formula (based on the whole formulation) is from 1 wt% to 30 wt%. The lipstick of the invention might also comprise pigments, pearl agents, water soluble ingredients, especially active ingredients, and other common ingredients.

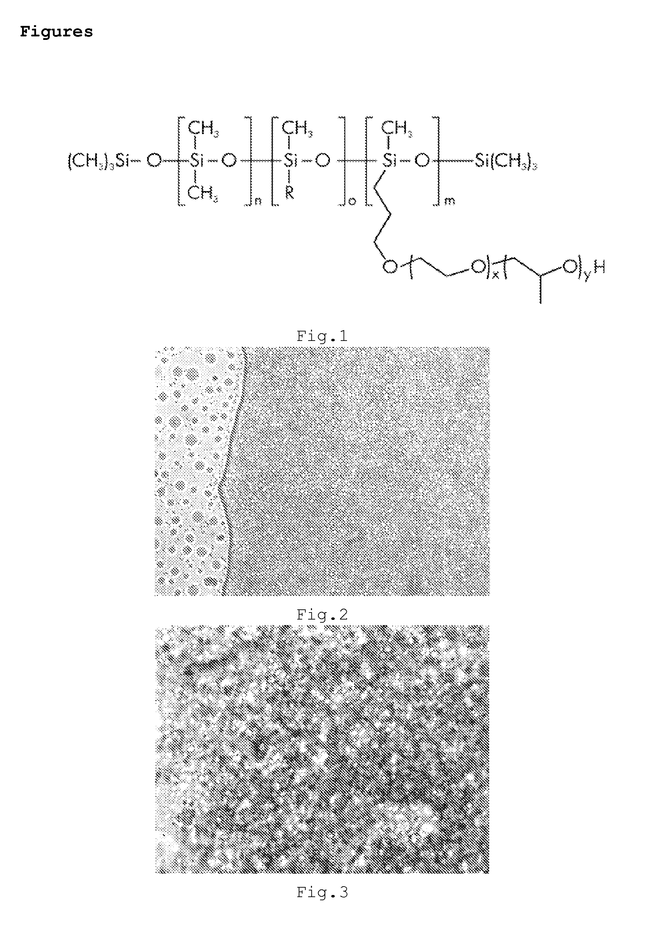

The emulsifiers used in the lipstick of the invention preferably contains a mixture of two or three components selected from poly glyceryl fatty esters, fatty esters and silicone derivative; preferable the mixture of two or three, preferably three components is selected from polyglyceryl-4 isostearate, cetyl PEG/PPG-10/1 silicone and hexyl laurate, wherein each component is from 25 wt% to 50 wt% of the whole emulsifier. Most preferable a product sold by Evonik Goldschmidt GmbH under the trade name ABIL® WE 09 is used as emulsifier. Figure 1 shows the chemical structure of the cetyl PEG/PPG-10/lsilicone in ABIL^{®} WE 09, the molecular weight of which is 14000g/mol, the radical R is a cetyl group, X = 10, Y = 1, n = 1 to 200, o = 1 to 100, m = 1 to 40. The other parts are polyglyceryl-4 isostearate (CAS No.91824-88-3) and hexyl laurate (CAS No. 34316-64-8).

The water phase in this invention may contain low molecular (having from 1 to 4 carbon atoms) hydrophilic ingredients, for example alcohols like glycerin, propylene glycol, 1,3-butylene glycol and ethanol.

It may also contain other water soluble ingredients, especially water soluble active ingredients like sodium hyaluronate, glucan, xanthan gum or guar gum, trimethylglycine (betain), zinc gluconate and magnesium gluconate.

The eutectic temperature of the oil components used in this invention is preferably from 30 °C to 55 °C. The oil components in this invention can for example be one or more of the following materials: microcrystalline wax, beewax, octyldodecanol (TEGOSOFT^{®} G20), myristyl myristate (TEGOSOFT^{®} MM), cetearyl alcohol (TEGO^{®} Alkanol 1618), stearyl heptanoate (TEGOSOFT^{®} SH), caprylic/capric triglyceride (TEGOSOFT^{®} CT), octyl methoxycinnamate, butyl methoxydibenzoylmethane, carnauba wax, jojoba oil, shea butter, castor oil and polyisobutene (REWOPAL^{®} PIB 1000). The products named in parenthesis are all available from Evonik Goldschmidt GmbH.

The lipstick of the invention might also comprise pigments. Examples for suitable pigments that might be used in the lip stick of the invention are: minerals, organic pigments like UNIPURE^{®} RED LC300, UNIPURE^{®} RED LC3075 or UNIPURE^{®} RED LC226 from the SENSIENT company. Preferably these pigments are first dispersed in the oil components like caprylic/capric triglyceride (TEGOSOFT^{®} CT) and polyisobutene (REWOPAL^{®} PIB 1000) to form a pigment pre dispersion. The pigments content in the whole composition is preferably of from 0.01 wt% to 2 wt%.

A pearl agent useful in this invention can be for example COVAPEARL SILVER 939 AS from SENSIENT Company. The pearl agent content in the whole composition is preferably of from 0.1 wt% to 5 wt%.

From 0.01 wt% to 0.5 wt% fragments could also be added into the composition if needed.

The above described high water content lipstick completely fits general lipstick quality standards. It is stable in a temperature range of from -15 °C to 45 °C for 3 months: stable means that the stick keeps shape and no oil separation, cracks, small holes or bubbles are observed. It also keeps its shape when applied and meets the requirements of easy and soft spreading at room temperature.

The high water content lipstick of the present invention might be produced by a process known in the prior art. Preferably the high water content lipstick of the present invention is obtained by the process of the invention as described below.

If the lipstick is prepared by normal process of mixing oil and water phase and emulsifying, a large amount of bubbles might be generated due to high speed of homogenizing. Therefore in this invention, a novel pre-emulsifying process is introduced: A W/O pre-emulsion is formed by mixing water phase, emulsifier and only a small part of the oil phase at first and then to mix the pre-emulsion with rest oil phase at a high temperature. This novel process can effectively reduce the bubbles.

The process of the invention comprises the following steps:
1. Preparing a W/O pre-emulsion at room temperature. A small part of the liquid oil phase, preferably 0.1 wt% to 10 wt%, preferably 0.5 wt% to 5 wt%, of the liquid oil phase and emulsifier are mixed at room temperature and stirred well, and then the water phase is added slowly while continuous stirring to obtain a white and shiny W/O pre-emulsion.
2. The remnant oil compositions are mixed and heated to a temperature of from 70 °C to 90 °C.
3. The W/O pre-emulsion is added under stirring to the above mixture of oil compositions still at a temperature of from 70 °C to 90 °C.
4. Molding the mixture at a temperature of from 70 °C to 90 °C, cooling down the mould to room temperature and storing the mould at a temperature of from -15 °C to -25 °C, for example in a refrigerator, preferably for at least 15 minutes, more preferably for 15 to 45 minutes, then releasing mould.

In the W/O pre-emulsion prepared by the above process, the weight ratio of oil phase to the emulsifiers is preferably from 1:10 to 10:1.

It might be advantageous to add pigments or pearl agents to the lip stick of the invention. In the process of the invention the pigments or pearl agents are preferably added after step 3 at a temperature of the mixture of from 70 °C to 90 °C while stirring.

It might be advantageous to add water soluble compounds, especially water soluble active agents to the lip stick of the invention. In the process of the invention the water soluble compounds, especially water soluble active agents are added, by dissolving them in the water phase prior to step 1.

The lipstick in the present invention could be any desired shape, preferable stick, wherein the diameter of it is preferably from 5 mm to 15 mm.

In this invention, according to the technical procedures described above, preferably the composition of polyglyceryl fatty ester, fatty ester and modified polysiloxane is used as emulsifier and a W/O pre-emulsion is formed at first and then the pre-emulsion is mixed with the oil phase. This technical process can increase the water content in lipsticks effectively. In the present invention, a lipstick with high water content stable during storage and application was obtained. The lip stick of the invention keeps its stick shape and shows no oil separation, crack, small holes and bubbles. The lipstick cost was reduced and furthermore, watersoluble active ingredients can be added into the lipstick and give the consumer a softer, comfortable and caring feeling when applied. The lip sticks of the invention can be applied in lip care or lip cosmetic products.

The lip sticks of the invention can, e.g., comprise at least one additional component chosen from the group consisting of
emollients
emulsifiers and surfactants
thickeners/viscosity regulators/stabilizers
UV/light screening agents
antioxidants
solids
pearlescent additives
insect repellents
preservatives
fragrances
colorants
biogenic active substances
care additives.

Preferred examples of these components are listed in DE 102005011785 and EP 2000124 which are incorporated by reference.

### Figures

The figures below give illustrate the invention without limiting the invention to the figures.
Figure 1 shows the chemical structure of Cetyl PEG/PPG-10/1 Dimethicone (ABIL^{®} WE 09).
Figure 2 shows the microscope photo of the lipstick in Example 1 with 17 % water content (10X10).
Figure 3 shows the microscope photo of the lipstick in Example 2 with 30 % water content (10X40).

The invention is described in detail by the following examples. The scope of the invention should not be bound to the embodiments of the examples but is given by the scope of the claims.

### Examples

### Example 1:

The lip sticks of the following Examples 1 were prepared from the components given in table 1 using the following steps:
1. Preparing a W/O pre-emulsion at room temperature:
   firstly, 5 wt% ABIL^{®} WE 09 and 2 wt% caprylic/capric triglyceride(TEGOSOFT^{®} CT) were mixed at room temperature and stirred well; then the water and glycerin were added slowly with continuous stirring to obtain a W/O pre-emulsion.
2. The remnant oil compositions were mixed and heated to a temperature of 70 °C.
3. The W/O pre-emulsion was added to the above oil composition mixture with the temperature of 85 °C while continuously stirring.
4. Molding at 80 °C, cooling down to room temperature and storing at -15 °C in a refrigerator for 45 minutes, then releasing the mould.

**Table 1: components used to form the lipstick of example 1**

| | Compositions | wt% |
|---|---|---|
| A | Ployglyceryl-4 Isostearate;Cetyl PEG/PPG-10/1Dimethicone; Hexyl Laurate(ABIL^{®} WE 09) | 5 |
| | Caprylic/Capric Triglyceride(TEGOSOFT^{®} CT) | 2 |
| B | Glycerin | 3 |
| | Water | 17.1 |
| C | Octyldodecanol(TEGOSOFT^{®} G20) | 18.7 |
| | Microcristalline Wax (Kahl, Microcristalline Wax 6089) | 15.1 |
| | Myristyl Myristate(TEGOSOFT^{®} MM) | 3.4 |
| | Cetearyl Alcohol(TEGO^{®} Alkanol 1618) | 1.7 |
| | Beeswax (Kahl, Beeswax 8044) | 0.4 |
| | Stearyl Heptanoate(TEGOSOFT^{®} SH) | 5.9 |
| | Octyl Methoxycinnamate | 2.5 |
| | Butyl Methoxydibenzoylmethane | 0.4 |
| | Carnauba(Kahl, Carnauba 2901) | 1 |
| | Caprylic/Capric Triglyceride(TEGOSOFT^{®} CT) | 13.8 |
| | Jojoba oil | 0.8 |
| | Shea Butter | 0.8 |
| | Castor oil | 8.4 |

| | | |
|---|---|---|
| A: W/O pre-emulsion; B: Water phase; C: Oil phase | | |

The product prepared according to Example 1 keeps its stick shape, 12mm in diameter, and shows no oil separation, crack, small holes and bubbles after 3 months storing at a temperature of -15 °C or 45 °C separately.

Fig. 2 shows the microscope figure of the lipstick with 20 wt% water phase (17 wt% pure water) in the magnification of 100. It indicates a well dispersed emulsion with homogeneous texture, well emulsified with fine particles and clear sample border.

### Example 2:

The lip sticks in example 2 were prepared from the components given in table 2 by the process described in example 1.

**Table 2: components used to form the lipstick of example 2**

| | Compositions | wt% |
|---|---|---|
| A | Ployglyceryl-4 Isostearate;Cetyl PEG/PPG-10/1Dimethicone; Hexyl Laurate(ABIL^{®} WE 09) | 5 |
| | Caprylic/Capric Triglyceride(TEGOSOFT^{®} CT) | 2 |
| B | Glycerin | 0 |
| | Water | 30 |
| C | Octyldodecanol(TEGOSOFT^{®} G20) | 13.8 |
| | Microcristalline Wax (Kahl, Microcristalline Wax 6089) | 15.0 |
| | Myristyl Myristate(TEGOSOFT^{®} MM) | 3.4 |
| | Cetearyl Alcohol(TEGO^{®} Alkanol 1618) | 1.7 |
| | Beeswax (Kahl, Beeswax 8044) | 0.4 |
| | Stearyl Heptanoate(TEGOSOFT^{®} SH) | 5.9 |
| | Octyl Methoxycinnamate | 2.5 |
| | Butyl Methoxydibenzoylmethane | 0.4 |
| | Carnauba(Kahl, Carnauba 2901) | 1.0 |
| | Caprylic/Capric Triglyceride(TEGOSOFT^{®} CT) | 8.8 |
| | Jojoba oil | 0.8 |
| | Shea Butter | 0.8 |
| | Castor oil | 8.5 |

| | | |
|---|---|---|
| A: W/O pre-emulsion; B: Water phase; C: oil phase | | |

Fig. 3 shows the microscope figure of the lipstick of example 2 with 30 wt% water phase in the magnification of 400. As shown in this figure, the inner structure of lipstick is homogeneous and the round particle of water phase is dispersed well.

### Example 3:

The lip sticks of the following example 3 were prepared from the components given in table 3 using the following process steps:
1. Preparing a W/O pre-emulsion at room temperature:
   firstly, 4.4 wt% ABIL^{®} WE 09 and 1.76 wt% TEGOSOFT^{®} CT were mixed at room temperature and
   stirred well; then the water and glycerin were added slowly with continuous stirring to obtain a W/O pre-emulsion.
2. The remnant oil compositions C were mixed and heated to a temperature of 90 °C.
3. The W/O pre-emulsion was added to the above oil composition mixture C with the temperature of 85 °C while continuously stirring.
4. The pigment phase D was heated and added to the mixture got from step 3. The obtained mixture was stirred at 90 °C. And the pearl agent E was added at last with tenderly stirring.
5. Molding at 7 0 °C, cooling down to room temperature and storing at -25 °C in a refrigerator for 15 minutes, then releasing the mould.

**Table 3: components used to form the lipstick of example 3**

| | Compositions | wt% | wt% |
|---|---|---|---|
| A | Ployglyceryl-4 Isostearate; Cetyl PEG/PPG-10/1 Dimethicone; Hexyl Laurate(ABIL^{®} WE 09) | 4.4 | 6.16 |
| | Caprylic/Capric Triglyceride(TEGOSOFT^{®} CT) | 1.76 | |
| B | Glycerin | 2.64 | 17.6 |
| | Water | 14.96 | |
| C | Octyldodecanol(TEGOSOFT^{®} G20) | 12.15 | 64.24 |
| | Microcristalline Wax (Kahl, Microcristalline Wax 6089) | 17.6 | |
| | Myristyl Myristate(TEGOSOFT^{®} MM) | 3.0 | |
| | Cetearyl Alcohol(TEGO^{®} Alkanol 1618) | 1.5 | |
| | Beeswax (Kahl, Beeswax 8044) | 0.35 | |
| | Stearyl Heptanoate(TEGOSOFT^{®} SH) | 5.19 | |
| | Octyl Methoxycinnamate | 2.2 | |
| | Butyl Methoxydibenzoylmethane | 0.35 | |
| | Carnauba(Kahl, Carnauba 2901) | 0.9 | |
| | Caprylic/Capric Triglyceride(TEGOSOFT^{®} CT) | 12.2 | |
| | Jojoba oil | 0.7 | |
| | Shea Butter | 0.7 | |
| | Castor oil | 7.4 | |
| D | Polyisobutene(REWOPAL^{®} PIB 1000) | 3.63 | 8.5 |
| | Caprylic/Capric Triglyceride(TEGOSOFT^{®} CT) | 3.63 | |
| | TiO₂ (pigment grade) | 0.73 | |
| | UNIPURE RED LC300 (SENSIENT) | 0.37 | |
| | UNIPURE RED LC3075(SENSIENT) | 0.07 | |
| | UNIPURE RED LC226(SENSIENT) | 0.07 | |
| E | COVAPEARL SILVER 939 AS(SENSIENT) | 3.5 | 3.5 |

| | | | |
|---|---|---|---|
| A: W/O pre-emulsion; B: Water phase; C: Oil phase; D: Pigments; E: Pearl agents | | | |

## Claims

1. A high water content lip stick, containing from 1 wt% to 10 wt% of emulsifiers, from 1 wt% to 30 wt% of a water phase and from 69 wt% to 98 wt% of an oil components phase, wherein the proportion of pure water of the whole formula is from 1 wt% to 30 wt%.

2. The lip stick according to claim 1, wherein the proportion of pure water of the whole formula is from 10 wt% to 30 wt%, preferably 17 wt% to 25 wt%.

3. The lip stick according to claim 1 or 2, wherein the emulsifier contains the mixture of two or three components selected from poly glyceryl fatty esters, fatty esters and silicone derivative.

4. The lip stick according to claim 3, wherein the emulsifier contains the mixture of two or three components selected from selected from polyglyceryl-4 isostearate, cetyl PEG/PPG-10/1 silicone and hexyl laurate, wherein each component is from 25 wt% to 50 wt% of the whole emulsifier.

5. The lip stick according to any of claims 1 to 4, wherein the water phase comprises only water or water and alcohols with 1 to 4 carbon atoms or water and polysaccharides and/or water soluble active ingredients.

6. The lip stick according to claim 5, wherein the alcohol is selected from glycerin, propylene glycol, 1,3-butylene glycol and ethanol.

7. The lip stick according to claim 5, wherein the water soluble polysaccharides and/or water soluble active ingredients are selected from sodium hyaluronate, glucan, xanthan gum, guar gum, trimethylglycine (betain), zinc gluconate and magnesium gluconate.

8. The lip stick according to any of the preceeding claims, wherein the eutectic temperature of the oil components is from 30 °C to 55 °C.

9. The lip stick according to any of the preceding claims, wherein the oil component phase comprises one or more of the compounds of the group comprising microcrystalline wax, beewax, octyldodecanol, myristyl myristate, cetearyl alcohol, stearyl heptanoate, caprylic/capric triglyceride, octyl methoxycinnamate, butyl methoxydibenzoylmethane, carnauba wax, jojoba oil, shea butter, castor oil, and polyisobutene.

10. The process for manufacturing the lip stick according to one of the preceding claims, including the steps:
1. preparation of a W/O pre-emulsion at room temperature, wherein 1 wt% - 10 wt% oil phase of the whole liquid oil phase and emulsifier are mixed at room temperature and stirred well, and then the water phase is added slowly with continuous stirring to obtain a white and shiny W/O pre-emulsion;
2. Mixing and Heating the remnant oil components to a temperature of from 70 °C to 90 °C;
3. Adding the W/O pre-emulsion to the oil components mixture of step 2 having a temperature of from 70 °C to 90 °C under stirring;
4. Molding the mixture at a temperature of from 70 °C to 90 °C, cooling down the molded mixture to room temperature and storing the mould at -15 °C to -25 °C, then releasing the mould.

11. The process according to claim 10, wherein the weight ratio of oil phase to emulsifier in the W/O pre-emulsion is between 1:10 and 10:1.

12. The process according to claim 6, wherein pigments and/or pearl agents are added to the mixture after step 3 and before step 4 at a temperature of from 70 °C to 90 °C while stirring.

13. The process according to claim 6, wherein water soluble compounds, solved in the water phase before adding the water phase in step 1.
